# EUROPEAN PATENT APPLICATION

(11) **EP 4 335 872 A1**
(43) Date of publication of application: **13.03.2024**
(21) Application number: 22799170.0
(22) Date of filing: 06.05.2022
(51) Int. Cl.: C07K 16/28, A61P 35/00, A61P 9/10, A61P 1/14, A61K 39/00

(54) **BISPECIFIC ANTIBODY SPECIFICALLY BINDING TO CD47 AND PD-L1**

(30) Priority: 07.05.2021 KR 20210059596
(71) Applicant: Immuneoncia Therapeutics, Inc., Yongin-si, Gyeonggi-do 17084 (KR)
(72) Inventor: KIM, Jeong Kook, Yongin-si, Gyeonggi-do 17084 (KR); JEON, A-Ra, Yongin-si, Gyeonggi-do 17084 (KR); YOO, Hyeon Seok, Seoul 06798 (KR); PARK, Jihyun, Yongin-si, Gyeonggi-do 17084 (KR); PARK, Ji Eun, Yongin-si, Gyeonggi-do 17084 (KR); CHOI, Ji-Hye, Yongin-si, Gyeonggi-do 17084 (KR); SHIN, Heewook, Yongin-si, Gyeonggi-do 17084 (KR); CHOI, Jiyea, Yongin-si, Gyeonggi-do 17084 (KR); SONG, Sun Kwang, Yongin-si, Gyeonggi-do 17084 (KR); KIM, Heung Tae, Yongin-si, Gyeonggi-do 17084 (KR); KIM, Sung Ho, Yongin-si, Gyeonggi-do 17084 (KR)
(74) Representative: Dehns
(86) International application number: PCT/KR2022/006521
(87) International publication number: WO 2022/235127

(57) **Abstract**

The present invention relates to a bispecific antibody. Having high binding affinity tumor cells expressing PD-L1 and/or CD47, the bispecific antibody of the present invention exhibits an excellent anti-tumoral effect with minimal side effects of hemagglutination.

## Description

### Technical Field

The present disclosure relates to a bispecific antibody. Specifically, the present disclosure relates to a bispecific antibody that specifically binds to CD47 and PD-L1, a use thereof, and a preparation method thereof.

### Background Art

Immune function in the body is regulated through co-stimulatory and co-inhibitory signals that occur simultaneously with antigen recognition. However, cancer cells change their tumor microenvironment or suppress immune function, thereby evading immune attacks. As one of such evasion strategies, cancer cells change immune checkpoint function to suppress function of immune cells. For example, cancer cells express specific cell surface proteins, such as PD-L1 or CD47, which bind to immune cells such as T cells or macrophages and suppress function of the immune cells.

As such mechanism is revealed, development of immune checkpoint inhibitors is actively conducted in which the inhibitors block activity of immune checkpoint proteins, such as the above-mentioned specific cell surface proteins, and activate immune cells to attack cancer cells. As PD-L1 inhibitors, various anti-PD-L1 monoclonal antibodies such as Tecentriq (atezolizumab), Imfinzi (durvalumab), and Bavencio (avelumab) are commercially available with FDA approval or are in clinical stages. In addition, several anti-CD47 monoclonal antibodies are being studied or are in clinical stages.

However, immune checkpoint inhibitors have problems such as insufficient patient response rates, adverse effects, and low bioavailability. Thus, development of new immune checkpoint inhibitors is still required.

### Disclosure of Invention

### Technical Problem

The present disclosure aims to solve all of the above-mentioned problems.

An object of the present disclosure is to provide a bispecific antibody that specifically binds to CD47 and PD-L1.

Another object of the present disclosure is to provide a use of a bispecific antibody that specifically binds to CD47 and PD-L1 for prevention or treatment of cancer.

Yet another object of the present disclosure is to provide a use of a bispecific antibody that specifically binds to CD47 and PD-L1 for manufacture of a medicament for preventing or treating cancer.

Still yet another object of the present disclosure is to provide a pharmaceutical composition comprising a bispecific antibody that specifically binds to CD47 and PD-L1.

Still yet another object of the present disclosure is to provide a method for preventing or treating cancer, comprising administering to a subject a bispecific antibody that specifically binds to CD47 and PD-L1.

Still yet another object of the present disclosure is to provide a polynucleotide, an expression vector, a host cell, or a method, for preparing a bispecific antibody that specifically binds to CD47 and PD-L1.

The object of the present disclosure is not limited to the objects mentioned above. The object of the present disclosure will become clearer from the following description and may be realized by the means and combinations thereof as set forth in the claims.

### Solution to Problem

A representative configuration of the present disclosure to achieve the object is as follows.

According to an aspect of the present disclosure, there is provided a bispecific antibody, comprising a first antigen binding domain that specifically binds to CD47 and a second antigen binding domain that specifically binds to PD-L1, wherein the first antigen binding domain comprises a heavy chain variable region and a light chain variable region, the heavy chain variable region comprising CDR1 that comprises the amino acid sequence of SEQ ID NO: 17, CDR2 that comprises the amino acid sequence of SEQ ID NO: 19, and CDR3 that comprises the amino acid sequence of SEQ ID NO: 21, and the light chain variable region comprising CDR1 that comprises the amino acid sequence of SEQ ID NO: 23, CDR2 that comprises the amino acid sequence of SEQ ID NO: 25, and CDR3 that comprises the amino acid sequence of SEQ ID NO: 27; and the second antigen binding domain comprises a heavy chain variable region and a light chain variable region, the heavy chain variable region comprising CDR1 that comprises the amino acid sequence of SEQ ID NO: 29, CDR2 that comprises the amino acid sequence of SEQ ID NO: 31, and CDR3 that comprises the amino acid sequence of SEQ ID NO: 33, and the light chain variable region comprising CDR1 that comprises the amino acid sequence of SEQ ID NO: 35, CDR2 that comprises the amino acid sequence of SEQ ID NO: 37, and CDR3 that comprises the amino acid sequence of SEQ ID NO: 39.

According to a certain embodiment, the bispecific antibody comprises a first antigen binding domain comprising a heavy chain variable region of SEQ ID NO: 1 and a light chain variable region of SEQ ID NO: 3, and a second antigen binding domain comprising a heavy chain variable region of SEQ ID NO: 5 and a light chain variable region of SEQ ID NO: 7.

According to a certain embodiment, the bispecific antibody comprises an Fc domain, a first antibody fragment (for example, Fab fragment) comprising the antigen binding domain that specifically binds to CD47, and a second antibody fragment (for example, scFv fragment) comprising the antigen binding domain that specifically binds to PD-L1.

According to a certain embodiment, in the bispecific antibody, the second antibody fragment (for example, scFv fragment) is fused, via a peptide linker, to the C-terminus of the Fc domain.

According to a certain embodiment, the bispecific antibody comprises an Fc domain, two Fab fragments each comprising the antigen binding domain that specifically binds to CD47, and two scFv fragments each comprising the antigen binding domain that specifically binds to PD-L1.

According to a certain embodiment, the bispecific antibody comprises a single chain variable fragment (scFv) comprising the antigen binding domain that specifically binds to PD-L1.

According to a certain embodiment, the bispecific antibody comprises a Fab fragment comprising the antigen binding domain that specifically binds to CD47.

According to a certain embodiment, the bispecific antibody comprises an Fc region derived from the heavy chain constant region (CH) of IgG1.

According to a certain embodiment, the Fc region of the bispecific antibody comprises heavy chain constant regions CH1, CH2, and CH3 derived from IgG1, and CH3 contains amino acid substitutions of E239D and M241L with respect to SEQ ID NO: 11. The Fc region may act on effector cells to exhibit an immune activation effect, in particular, an increased immune activation effect.

According to a certain embodiment, the bispecific antibody comprises an antibody fragment comprising the antigen binding domain that specifically binds to PD-L1, wherein the antibody fragment is fused, via a peptide linker, to the C-terminus of the Fc domain.

According to a certain embodiment, the single chain variable fragment (scFv) of the bispecific antibody comprises the amino acid sequence of SEQ ID NO: 9.

According to a certain embodiment, the Fc region of the bispecific antibody comprises the amino acid sequence of SEQ ID NO: 11.

According to a certain embodiment, the bispecific antibody specifically binds to cancer cells that express PD-L1, CD47, or both.

According to another aspect of the present disclosure, there is provided a use of the bispecific antibody of the present disclosure for prevention or treatment of various carcinomas, including breast cancer, lung cancer, B cell-derived lymphoma, and T cell-derived lymphoma.

According to yet another aspect of the present disclosure, there is provided a pharmaceutical composition for prevention or treatment of various carcinomas, including breast cancer, lung cancer, B cell-derived lymphoma, and T cell-derived lymphoma, the pharmaceutical composition comprising the bispecific antibody of the present disclosure.

According to still yet another aspect of the present disclosure, the bispecific antibody of the present disclosure may be used in combination with chemotherapy, radiation therapy, and/or another cancer immunotherapeutic agent.

According to still yet another aspect of the present disclosure, there is provided a method for preventing or treating various carcinomas, including breast cancer, lung cancer, B cell-derived lymphoma, and T cell-derived lymphoma, comprising administering, to a subject in need thereof, an effective amount of the bispecific antibody of the present disclosure.

According to still yet another aspect of the present disclosure, there is provided a method for inhibiting growth of tumor cells in a subject, comprising administering to the subject having tumor cells an effective amount of the bispecific antibody of the present disclosure.

According to still yet another aspect of the present disclosure, there is provided a polynucleotide encoding the bispecific antibody of the present disclosure.

According to still yet another aspect of the present disclosure, there is provided a host cell that comprises a polynucleotide encoding the bispecific antibody of the present disclosure.

According to still yet another aspect of the present disclosure, there is provided a method for preparing the bispecific antibody of the present disclosure, comprising culturing a host cell that comprises a polynucleotide encoding the bispecific antibody under a condition suitable for expression of the bispecific antibody, and collecting the bispecific antibody from the culture.

### Advantageous Effects of Invention

According to the present disclosure, the bispecific antibody that specifically binds to CD47 and/or PD-L1 can be effectively used for prevention or treatment of various carcinomas, including breast cancer, lung cancer, B cell-derived lymphoma, and T cell-derived lymphoma. Specifically, the bispecific antibody of the present disclosure has excellent antigen binding ability, in particular, significantly high binding ability to a CD47 antigen, as compared with anti-CD47 antibodies and/or anti-PD-L1 antibodies, while maintaining low binding ability to red blood cells (RBCs). For this reason, it was identified that the bispecific antibody of the present disclosure exhibited an excellent anti-tumor effect due to its high binding ability to tumor cells expressing CD47 and/or PD-L1, while exhibiting a minimal side effect of hemagglutination due to its low binding ability to RBCs. In addition, the bispecific antibody of the present disclosure exhibits a cancer suppression effect on cells expressing PD-L1 and/or CD47 and an effect of activating NK cells and T cells, as shown in evaluation of its antibody dependent cellular cytotoxicity (ADCC) activity. Furthermore, it has been proven that the bispecific antibody of the present disclosure exhibits an inhibitory effect on cancer growth in a breast cancer cell line xenograft model using BALB/c nude mice that lack adaptive immune cells, and a CT26-hPD-L1/hCD47 cell line colorectal cancer syngeneic model using BALB/c-hPD-1/hSIRPα mice, and also exhibits an inhibitory effect on tumor re-challenge due to generation of memory T cells. Therefore, the bispecific antibody according to the present disclosure can be effectively used for prevention, amelioration, or treatment of various carcinomas, including breast cancer, lung cancer, B cell-derived lymphoma, and T cell-derived lymphoma, through its cancer growth inhibition effect and immune cell activation effect.

### Brief Description of Drawings

FIG. 1 illustrates a schematic diagram of a bispecific antibody against CD47 and PD-L1, according to the present disclosure.
FIG. 2 illustrates results obtained by subjecting the antibody prepared in Example 1.2 to SDS-PAGE analysis with 4% to 20% denaturation under reducing and non-reducing conditions. Molecular weight markers are expressed in kilodalton.
FIG. 3 illustrates an analytical gel-filtration elution profile observed for the antibody prepared in Example 1.2 (column: Superdex 200 increase 10/300 GL; absorption wavelength: 214 nm; buffer: phosphate buffered saline (PBS); temperature (°C): 19; flow rate (ml/min): 0.75; SOP/WI used: WI PP 24.0; lot number: 120520).
FIGS. 4A and 4B illustrate results obtained by analyzing, with ELISA, binding capacity to single and dual ligands, respectively, of the bispecific antibody against CD47 and PD-L1.
FIG. 5 illustrates binding capacity of the bispecific antibody to PD-L1 enriched tumor cells and CD47 enriched tumor cells, respectively.
FIGS. 6A to 6D illustrate binding capacity of the bispecific antibody to WT, PD-L1 knockout (KO), CD47 KO, and PD-L1/CD47 double KO cells of MDA-MB-231, respectively.
FIG. 7 illustrates results obtained by subjecting the bispecific antibody to an RBC binding assay.
FIG. 8 illustrates results obtained by subjecting the bispecific antibody to a hemagglutination assay.
FIG. 9 illustrates phagocytosis results of the bispecific antibody on four types of cells (MDA-MB-231, H1975, Raji, and SR786).
FIGS. 10A and 10B illustrate results obtained by subjecting the bispecific antibody to an ADCC NFAT-Luc assay in Raji cells and MDA-MB-231 cells, respectively.
FIG. 11 illustrates results obtained by subjecting the bispecific antibody to an ADCC assay of NK cells in MDA-MB-231 cells.
FIG. 12 illustrates results obtained by subjecting the bispecific antibody to MLR analysis to identify its T cell activation effect.
FIG. 13 illustrates sites of tumor inoculation in the mice mentioned in Examples 3.1 to 3.3.
FIG. 14 illustrates results of efficacy study obtained by identifying inhibition of cancer growth, which is caused by the bispecific antibody, in BALB/c nude mice transplanted with human MDA-MB-231 cell line.
FIG. 15 illustrates results of efficacy study obtained by identifying inhibition of cancer growth, which is caused by the bispecific antibody, in CT26-hPD-L1/hCD47 tumor allograft BALB/c-hPD-1/hSIRPα mice.
FIG. 16 illustrates a tumor volume curve with treatment of a CT26-hPD-L1/hCD47 tumor allograft model in BALB/c-hPD-1/hSIRPα mice, in connection with the re-challenge study of Example 3.3. Data are presented as mean ± SEM.

### Best Mode for Carrying out Invention

The detailed description of the present disclosure set forth below will be described with reference to specific drawings with respect to specific embodiments in which the present disclosure may be practiced. However, the present disclosure is not limited thereto and, if appropriately described, is limited only by the appended claims, along with all equivalents as claimed by those claims. Technical and scientific terms used in the present specification have the same meaning as generally used in the art to which the present disclosure pertains, unless otherwise defined. For purposes of interpreting the present specification, the following definitions will apply, and terms used in the singular will include the plural where appropriate and vice versa.

### Definition

As used herein, the term "antigen binding molecule" refers to a molecule that specifically binds to an antigenic determinant. Examples of the antigen binding molecule include, but are not limited to, antibodies, antibody fragments, and scaffold antigen binding proteins.

The term "antibody" is used in the broadest sense and includes a variety of antibody structures, including but not limited to monoclonal antibodies, polyclonal antibodies, monospecific or multispecific antibodies (for example, bispecific antibodies), antibody fragments having antigen-binding activity, and antibody fusions (for example, a fusion of an antibody with a (poly)peptide or a fusion of an antibody with a compound). As used herein, the prefix "anti-," when it relates to an antigen, means that the antibody in question is reactive with that antigen. Antibodies reactive with a particular antigen may be produced by synthetic and/or recombinant methods, such as screening of recombinant antibody libraries in phage or similar vectors, or by immunization of animals with antigens or antigen-encoding nucleic acids; however, the present disclosure is not limited thereto. A representative IgG antibody consists of two identical heavy chains and two identical light chains, held together by disulfide bonds. Each of the heavy and light chains comprises a constant region and a variable region. Each of the heavy chain variable region (HVR) and light chain variable region (LVR) contains three segments called "complementarity determining regions" ("CDRs") or "hypervariable regions," which are mainly involved in binding to an antigenic epitope. The CDRs are numbered sequentially from the N-terminus and are commonly referred to as CDR1, CDR2, and CDR3. A more conserved region in the variable region, which is located outside the CDRs, is referred to as "framework region" ("FR"). In the present specification, the antibody may be, for example, an animal antibody, a chimeric antibody, a humanized antibody, or a human antibody.

The term "monoclonal antibody" refers to an antibody obtained from a population of substantially homogeneous antibodies. That is, individual antibodies included in the population are identical to each other and/or bind to the same epitope, except for possible variant antibodies (for example, antibodies with naturally occurring mutations during monoclonal antibody production) which are present in minor amounts. In contrast to polyclonal antibody preparations, which typically include different antibodies directed against different determinants (epitopes), each monoclonal antibody of a monoclonal antibody preparation is directed against a single determinant on an antigen.

The term "monospecific" antibody refers to an antibody that has one or more binding sites each of which binds to the same epitope of the same antigen. The term "bispecific" means that an antigen binding molecule is capable of specifically binding two or more distinct antigenic determinants. Typically, a bispecific antigen binding molecule contains two antigen binding sites each of which is specific for a different antigenic determinant. In some embodiments, the bispecific antigen binding molecule is capable of binding to two antigenic determinants, such as two antigenic determinants expressed on two distinct cells, at the same time. In addition, the bispecific antigen binding molecules described herein may form part of a multispecific antibody.

The term "antibody fragment" refers to a portion of an antibody, which has specific binding ability to an antigen, or a polypeptide comprising the same. Examples of the antibody fragment include, but are not limited to, Fv, Fab, Fab', Fab'-SH, F(ab')2, diabodies, triabodies, tetrabodies, cross-Fab fragments, linear antibodies, single-chain antibody molecules (for example, scFv), and multispecific antibodies formed from antibody fragments and single domain antibodies.

The term "single chain variable fragment" or "scFv" refers to a fusion protein in which the variable regions of the heavy (VH) and light (VL) chains of an immunoglobulin are covalently linked to form a VH-VL heterodimer. The heavy chain (VH) and light chain (VL) are connected directly or via a peptide linker to each other, in which the peptide linker connects the N-terminus of VH to the C-terminus of VL, or connects the C-terminus of VH to the N-terminus of VL.

The term "antigen binding domain" refers to a portion of an antigen binding molecule which specifically binds to an antigenic determinant. The antigen binding domain may be provided by, for example, at least one variable region. Preferably, the antigen binding domain may comprise an antibody light chain variable region and an antibody heavy chain variable region.

The term "Fc region" refers to the C-terminal region of an immunoglobulin heavy chain, which may be a homodimer consisting of a part of the hinge region, a CH2 domain, and a CH3 domain. The Fc region represents an intact antibody (for example, part of an IgG) produced by digestion with the enzyme papain. In an embodiment, the "Fc region" may be a native sequence Fc region or a variant Fc region. The "native sequence Fc region" refers to an amino acid sequence identical to that of an Fc region commonly found in nature. In the present disclosure, the Fc region may be an extended Fc region containing a part or the entirety of the heavy chain constant region 1 (CH1) and/or the light constant region 1 (CL1), excluding the variable regions of the heavy and light chains of an immunoglobulin, as long as the Fc region has substantially the same or improved effect as with its native type. In addition, the Fc region may be a region from which a significantly long partial amino acid sequence corresponding to CH2 and/or CH3 is removed. For example, in the present disclosure, the Fc region may be 1) CH1, CH2, and CH3, 2) CH1 and CH2, 3) CH1 and CH3, 4) CH2 and CH3, 5) a combination between one or two or more domains among CH1 to CH3 and an immunoglobulin hinge region (or a part of the hinge region), or 6) a dimer between each domain of the heavy chain constant region and the light chain constant region. In addition, in the present disclosure, the Fc region includes not only its native amino acid sequence but also sequence variants thereof. The amino acid sequence variant means that deletion, insertion, non-conservative or conservative substitution, or a combination thereof has occurred in at least one amino acid residue of a native amino acid sequence, causing the native amino acid sequence to have a different sequence.

The term "CD47" refers to a protein that is known to be present on the surface of cancer cells and is known to bind to SIRPα on macrophages and send a "do-not-eat-me" signal, thereby blocking phagocytosis of macrophages.

The term "PD-L1" refers to a protein that is known to be present on the surface of cancer cells and binds to PD-1, which is a protein on the surface of T cells, thereby preventing T cells from attacking cancer cells.

The term "linker" may refer to a peptide linker of 1 to 100 amino acids, specifically 2 to 50 amino acids, and more specifically 5 to 30 amino acids in length. The linker may include, for example, one or more amino acids selected from the group consisting of Gly, Asn, Ser, Thr, Ala, Asp, and the like; however, the present disclosure is not limited thereto. As an example, the linker may be represented by (GGGGS)n, where n is the number of repetitions of the unit (GGGGS) and may be 1 to 10, and specifically 1 to 5, in consideration of efficacy of a bispecific antibody. As another example, the linker may be a peptide fragment known to be capable of linking antibody fragment domains. For example, the linker may have an amino acid sequence of GGGGSGGGGSGGGGS.

The terms "polynucleotide," "oligonucleotide," and "nucleic acid" are used interchangeably and include DNA molecules (for example, cDNA or genomic DNA), RNA molecules (for example, mRNA), analogs of DNA or RNA produced using nucleotide analogs (for example, peptide nucleic acids and non-naturally occurring nucleotide analogs), and hybrids thereof. A nucleic acid molecule may be single-stranded or double-stranded. In an embodiment, the nucleic acid molecule of the present disclosure comprises a contiguous open reading frame that encodes an antibody, or a fragment, derivative, mutein, or variant thereof.

The term "subject" is used interchangeably with "patient" and may be a mammal, such as primates (for example, humans), companion animals (for example, dogs, cats, and the like), and livestock (for example, cattle, pigs, horses, sheep, goats, and the like), and laboratory animals (for example, rats, mice, guinea pigs, and the like), in need of prevention or treatment of cancer. In an embodiment of the present disclosure, the subject is a human.

The term "treatment" generally means obtaining a desired pharmacological and/or physiological effect. Such an effect has a therapeutic effect in that it partially or completely cures a disease and/or adverse effects caused by the disease. Desirable therapeutic effects include, but are not limited to, preventing occurrence or recurrence of a disease, alleviation of symptoms, diminishment of any direct or indirect pathological consequences of a disease, preventing metastasis, decreasing a rate of disease progression, amelioration or palliation of a disease state, and remission or improved prognosis. Preferably, "treatment" may mean medical intervention for an existing disease or disorder.

The term "prevention" means prophylactic treatment, that is, obtaining an effect of preventing, rather than treating, a disease. The "prevention" means obtaining a desired prophylactic pharmacological and/or physiological effect with a view to partially or completely preventing a disease or symptoms thereof.

The term "administration" means providing a subject with a substance (for example, the bispecific antibody of the present disclosure) to achieve a prophylactic or therapeutic purpose.

### Bispecific antibody of present disclosure

The present disclosure provides a novel bispecific antibody comprising a first antigen binding domain that specifically binds to CD47 and a second antigen binding domain that specifically binds to PD-L1. The antibody is capable of specifically binding to cancer cells expressing CD47, PD-L1, or both, and has particularly advantageous characteristics, for example, in terms of binding affinity, biological activity, immune cell activation, targeting efficacy, inhibition ability against tumor re-challenge, and decreased adverse effects.

According to an aspect of the present disclosure, there is provided a bispecific antibody, comprising a first antigen binding domain that specifically binds to CD47 and a second antigen binding domain that specifically binds to PD-L1, wherein the first antigen binding domain comprises a heavy chain variable region and a light chain variable region, the heavy chain variable region comprising CDR1 that comprises the amino acid sequence of SEQ ID NO: 17, CDR2 that comprises the amino acid sequence of SEQ ID NO: 19, and CDR3 that comprises the amino acid sequence of SEQ ID NO: 21, and the light chain variable region comprising CDR1 that comprises the amino acid sequence of SEQ ID NO: 23, CDR2 that comprises the amino acid sequence of SEQ ID NO: 25, and CDR3 that comprises the amino acid sequence of SEQ ID NO: 27; and the second antigen binding domain comprises a heavy chain variable region and a light chain variable region, the heavy chain variable region comprising CDR1 that comprises the amino acid sequence of SEQ ID NO: 29, CDR2 that comprises the amino acid sequence of SEQ ID NO: 31, and CDR3 that comprises the amino acid sequence of SEQ ID NO: 33, and the light chain variable region comprising CDR1 that comprises the amino acid sequence of SEQ ID NO: 35, CDR2 that comprises the amino acid sequence of SEQ ID NO: 37, and CDR3 that comprises the amino acid sequence of SEQ ID NO: 39.

In some embodiments, the first antigen binding domain that specifically binds to CD47 may comprise a heavy chain variable region of SEQ ID NO: 1 and a light chain variable region of SEQ ID NO: 3, and/or the second antigen binding domain that specifically binds to PD-L1 may comprise a heavy chain variable region of SEQ ID NO: 5 and a light chain variable region of SEQ ID NO: 7. In particular, the second antigen binding domain that specifically binds to PD-L1 may comprise a single chain variable fragment (scFv) of SEQ ID NO: 9.

In some embodiments, the bispecific antibody of the present disclosure may comprise an Fc domain, a first antibody fragment comprising the antigen binding domain that specifically binds to CD47, and a second antibody fragment comprising the antigen binding domain that specifically binds to PD-L1.

In some embodiments, the first antibody fragment may be a Fab fragment.

In some embodiments, the second antibody fragment may be a single chain variable fragment (scFv).

In some embodiments, the Fc region may act on effector cells to exhibit an immune activation effect. Preferably, the Fc region may be derived from the heavy chain constant region (CH) of IgG1. More preferably, the Fc region may comprise heavy chain constant regions CH1, CH2, and CH3 derived from IgG1, or heavy chain constant regions CH2 and CH3 derived from IgG1. Most preferably, CH3 of the Fc region may contain amino acid substitutions of E239D and M241L with respect to SEQ ID NO: 11.

In some embodiments, the Fc region may comprise the amino acid sequence of SEQ ID NO: 11.

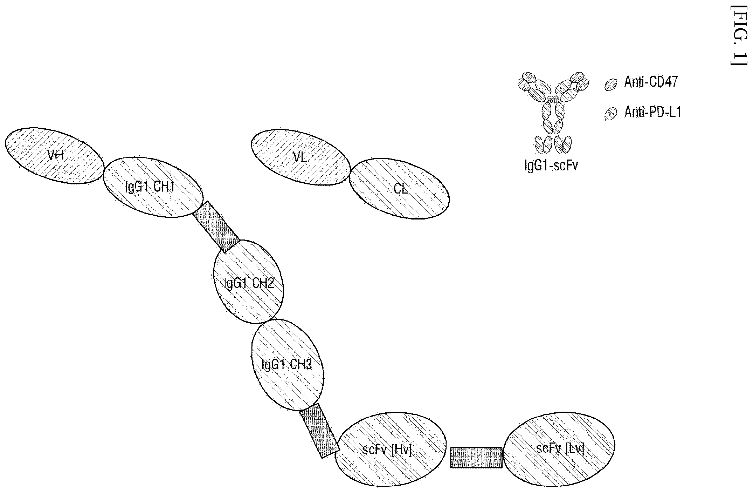

In some embodiments, the second antibody fragment may be fused to the C-terminus of the Fc region. More specifically, the second antibody fragment (that is, the N-terminus of the second antibody fragment) may be fused, via the heavy chain variable region, to the C-terminus of the Fc region (see FIG. 1). Here, the second antibody fragment may be fused, via a linker, to the C-terminus of the Fc region. The linker may be a peptide linker and may have, for example, an amino acid sequence of GGGGS, GGGGSGGGGSGGGGS, or GSGSGSGSGSGSGSGSGS.

In some embodiments, the second antibody fragment may comprise a variable region (for example, a single chain variable fragment (scFv)) in which a heavy chain variable region and a light chain variable region are connected to each other via a linker. As an example, the linker may be a peptide linker represented by (GGGGS)n, where n is the number of repetitions of the (GGGGS) unit, and may be 1 to 10, and specifically 1 to 5 in consideration of efficacy of a bispecific antibody. Preferably, the second antibody fragment may be an scFv fragment. More preferably, the scFv fragment may comprise the amino acid sequence of SEQ ID NO: 9.

In some embodiments, the bispecific antibody of the present disclosure may comprise an Fc domain, two Fab fragments each comprising the antigen binding domain that specifically binds to CD47, and two scFv fragments each comprising the antigen binding domain that specifically binds to PD-L1. Here, for each scFv fragment, its heavy chain variable domain may be fused, via a peptide linker, to the C terminus of the Fc domain (which is connected to the heavy chain of each Fab fragment).

In some embodiments, the bispecific antibody of the present disclosure may comprise a full-length antibody that specifically binds to CD47 and comprises two antibody heavy chains and two antibody light chains, and an antibody fragment (such as an scFv fragment) that specifically binds to PD-L1 and comprises a heavy chain variable region and a light chain variable region, wherein the heavy chain variable region of the antibody fragment may be fused directly or via a peptide linker to the C-terminus of the heavy chain of the full-length antibody.

In some embodiments, the bispecific antibody of the present disclosure may comprise a sequence having a sequence identity of at least 80%, preferably at least 90%, more preferably at least 95%, and most preferably at least 98% with the CDR sequences, the heavy chain variable region sequence, and/or the light chain variable region sequence.

In certain embodiments, an amino acid sequence variant of the bispecific antibody of the present disclosure is contemplated. For example, it may be desirable to improve binding affinity and/or other biological properties of the antibody. The amino acid sequence variant of the antibody may be prepared by introducing appropriate modifications into a nucleotide sequence encoding the molecule or by peptide synthesis. Such modifications include, for example, deletion of residues from, and/or insertion of residues into, and/or substitution of residues within, the amino acid sequence of the antibody. Any combination of various changes, including deletion, insertion, and substitution, may be made to arrive at a final construct, provided that the final construct possesses desired properties, such as antigen-binding property. Sites of interest for substitutional mutagenesis include heavy chain variable regions (HVRs) and framework regions (FRs). Conservative substitutions are provided in Table 1 under the heading "Preferred substitution" and are further described below in reference to amino acid side chain classes (1) to (6). Amino acid substitutions may be introduced into molecules of interest and the products screened for desired activity, for example, retained/improved antigen binding, decreased immunogenicity, or improved ADCC or CDC.

**[Table 1]**

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Ile |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; norleucine | Leu |

Amino acids may be grouped according to common side-chain properties as follows:
(1) hydrophobic: norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions involve exchanging a member of one of these classes for that of another class.

As used herein, the term "amino acid sequence variant" includes substantial variants in which amino acid substitution(s) exists in one or more hypervariable region residues of a parent antigen binding molecule (for example, a humanized or human antibody). In general, the resulting variant(s) selected for further study will have modifications (for example, improvements) in certain biological properties (for example, increased affinity and decreased immunogenicity) relative to its parent antigen binding molecule and/or will substantially retain certain biological properties of its parent antigen binding molecule. The exemplary substitutional variant is an affinity matured antibody, which may be conveniently produced, for example, using phage display-based affinity maturation techniques known in the art. Briefly, one or more HVR residues are mutated, and variant antigen binding molecules are displayed on phage and screened for particular biological activity (for example, binding affinity). In certain embodiments, substitutions, insertions, or deletions may occur within one or more HVRs so long as such alterations do not substantially reduce the ability of the antigen binding molecule to bind an antigen. For example, conservative alterations (for example, conservative substitutions as provided herein), which do not substantially reduce binding affinity, may be made in HVRs.

Amino acid sequence insertions may include amino- and/or carboxyl-terminal fusions of from one residue to polypeptides containing one hundred or more residues, as well as intrasequence insertions of single or multiple amino acid residues. Examples of the terminal insertion include antibodies with an N-terminal methionyl residue. Other insertional variants of the molecule may include fusions of a polypeptide, which increases a serum half-life of the antibody, to the N-terminus or C-terminus. In addition, other insertional variants of the molecule may include fusions of a polypeptide, which facilitates passage of the blood-brain barrier (BBB), to the N-terminus or C-terminus.

### Nucleic acid, vector, host cell, and preparation method

The bispecific antibody of the present disclosure may be prepared by any antibody production technique known in the art.

According to another aspect of the present disclosure, there is provided an isolated nucleic acid (for example, polynucleotide) encoding the bispecific antibody described herein or a fragment thereof. Such a nucleic acid may encode an amino acid sequence comprising the heavy chain variable region or heavy chain CDR regions of the bispecific antibody and/or an amino acid sequence comprising the light chain variable region or light chain CDR regions of the bispecific antibody.

In some embodiments, the bispecific antibody of the present disclosure may be expressed by a single polynucleotide encoding the entire antigen binding molecule or by a plurality of polynucleotides that are co-expressed. The polypeptides encoded by the plurality of polynucleotides may be linked to each other, for example, via disulfide bonds or other means for forming a functional antigen binding molecule. For example, the light chain portion of an immunoglobulin may be encoded by a polynucleotide isolated from the heavy chain portion of the immunoglobulin. In a case of being co-expressed, the heavy chain polypeptide will combine with the light chain polypeptide to form the immunoglobulin.

In an embodiment, the polynucleotide may comprise the sequence of SEQ ID NO: 2 and the sequence of SEQ ID NO: 4. In addition, the polynucleotide sequence may comprise the sequence of SEQ ID NO: 12.

In another embodiment, the polynucleotide may comprise the sequence of SEQ ID NO: 6 and the sequence of SEQ ID NO: 8. In addition, the polynucleotide sequence may comprise the sequence of SEQ ID NO: 10.

In yet another embodiment, the polynucleotide may be or comprise the sequence of SEQ ID NO: 15 and/or the sequence of SEQ ID NO: 16.

In still yet another embodiment, the polynucleotide may comprise a nucleic acid sequence having an identity of at least about 70%, at least about 75%, at least about 80%, at least about 85%, at least about 90%, at least about 95%, at least about 98%, or about 100% with the polynucleotide encoding the bispecific antibody of the present disclosure.

In still yet another embodiment, the polynucleotide may encode a polypeptide included in the bispecific antibody according to the present disclosure.

According to still yet another aspect of the present disclosure, there are provided one or more vectors (for example, expression vectors) containing the nucleic acid. The term "vector" refers to a nucleic acid that can be used to introduce another nucleic acid linked thereto into a cell. One type of vector is a "plasmid" that refers to a linear or circular double-stranded DNA molecule to which an additional nucleic acid segment can be ligated. Another type of vector is a viral vector (for example, replication-deficient retrovirus, adenovirus, and adeno-associated virus), in which case an additional DNA segment can be introduced into its viral genome. Certain vectors are capable of replicating themselves within host cells into which they are introduced (for example, bacterial vectors containing a bacterial origin of replication and episomal mammalian vectors). Other vectors (for example, non-episomal mammalian vectors) are integrated into the genome of a host cell, when introduced into the host cell, and replicate along with the host genome. The "expression vector" is a type of vector that can induce expression of a selected polynucleotide.

According to still yet another aspect of the present disclosure, there is provided a host cell that comprises one or more polynucleotides encoding the bispecific antibody of the present disclosure. The host cell may be a prokaryotic or eukaryotic cell. For example, the host cells may be a Chinese hamster ovary (CHO) cell or lymphoid cell. In a case where glycosylation or the like is not required, the antibody may be produced in bacteria.

According to still yet another aspect of the present disclosure, there is provided a method for preparing the bispecific antibody of the present disclosure, comprising culturing the host cell under a condition suitable for expression of the bispecific antibody of the present disclosure, and collecting the bispecific antibody from the culture.

For recombinant production of the bispecific antibody, for example, a polynucleotide encoding the above-described bispecific antibody is isolated and inserted into one or more vectors for further cloning and/or expression in a host cell. Such a nucleic acid may be isolated or sequenced using conventional procedures known in the art.

The method of culturing the transformed cells may be performed using methods well known in the art. For example, the bispecific antibody may be produced in large quantities by culturing the transformant in a nutrient medium, and the medium and culture conditions may be appropriately selected depending on a host cell. Conditions such as temperature, pH of a medium, and incubation time may be appropriately adjusted to be suitable for cell growth and mass production of a protein during culture.

To recover the bispecific antibody, any method known in the art for purification of the immunoglobulin, such as chromatography (ion exchange, affinity (for example, protein A), size exclusion, and the like), centrifugation, and differential solubility, may be used, or any other standard technique for protein purification may be used.

### Prophylactic or therapeutic method

According to still yet another aspect of the present disclosure, the bispecific antibody of the present disclosure may be used in a prophylactic or therapeutic method.

In some embodiments, the disease to be treated may be a proliferative disorder, in particular cancer. Specifically, the bispecific antibody of the present disclosure may be used to inhibit proliferation, survival, metastasis, and recurrence of cancer.

In some embodiments, there is provided a method for inhibiting growth of tumor cells in a subject, comprising administering to the subject having tumor cells an effective amount of the bispecific antibody of the present disclosure. In some embodiments, there is provided a method for preventing or treating cancer, comprising administering to a subject in need thereof an effective amount of the bispecific antibody of the present disclosure.

In some embodiments, the cancer may be solid cancer or hematological cancer.

In some embodiments, the cancer may be selected from the group consisting of ovarian cancer, colon cancer, colorectal cancer, breast cancer, lung cancer, myeloma, neuroblast-derived CNS tumor, monocytic leukemia, B cell-derived leukemia, T cell-derived leukemia, B cell-derived lymphoma, T cell-derived lymphoma, and mast cell-derived tumor, but is not limited thereto. Preferably, the cancer may be any one selected from the group consisting of ovarian cancer, lung cancer, B cell-derived lymphoma, and T cell-derived lymphoma.

The bispecific antibody according to the present disclosure may be administered in several ways depending on whether local or systemic treatment is desired and on the area to be treated. A method of administering the bispecific antibody to a subject may vary depending on purpose of administration, site of onset of disease, condition of the subject, and the like. The route of administration may be parenteral, by inhalation, topical, or local (for example, intralesional administration). For example, parenteral administration may include, but is not limited to, intravenous, subcutaneous, intraperitoneal, intrapulmonary, intraarterial, intramuscular, rectal, intravaginal, intraarticular, intraprostatic, intranasal, intraocular, intravesical, intrathecal, or intraventricular administration (for example, intracerebroventricular administration). In addition, in a case of being used in combination, the bispecific antibody of the present disclosure and an additional cancer immunotherapeutic agent may be administered by the same route or by different routes.

In the above-described method, the effective amount of the bispecific antibody according to the present disclosure may vary depending on age, gender, and body weight of the individual (patient), and is generally such that about 0.01 mg to 100 mg, or 5 mg to about 50 mg per kg of body weight may be administered once a day or in divided doses several times a day. However, since the effective amount may increase or decrease depending on route and period of administration, severity of disease, gender, body weight, age, and the like, the scope of the present disclosure is not limited thereto.

In some embodiments, the bispecific antibody of the present disclosure may be used or administered in combination with chemotherapy, radiation therapy, and/or another cancer immunotherapeutic agent (for example, anti-PD-1, anti-CTLA-4 antibodies, and the like). This combination therapy includes combined administration (administration in which two or more therapeutic agents are included in the same or separate compositions) and separate administration. In a case of the separate administration, administration of the bispecific antibody may occur prior to, concurrently with, and/or subsequent to administration/use of the additional therapeutic agent/therapy.

### Pharmaceutical composition

According to still yet another aspect of the present disclosure, there is provided a pharmaceutical composition, comprising the bispecific antibody of the present disclosure. The bispecific antibody may be included in the composition in a prophylactically or therapeutically effective amount.

In some embodiments, the pharmaceutical composition may be administered to a subject to inhibit proliferation, survival, metastasis, and recurrence of cancer. Accordingly, the pharmaceutical composition may be administered to a subject for prevention or treatment of cancer. Here, the pharmaceutical composition may be used or administered in combination with chemotherapy, radiation therapy, and/or another cancer immunotherapeutic agent (for example, anti-PD-1, anti-CTLA-4 antibodies, and the like). This combination therapy includes combined administration (administration in which two or more therapeutic agents are included in the same or separate compositions) and separate administration. In a case of the separate administration, administration of the bispecific antibody may occur prior to, concurrently with, and/or subsequent to administration/use of the additional therapeutic agent/therapy.

In some embodiments, the cancer may be solid cancer or hematological cancer.

In some embodiments, the cancer may be selected from the group consisting of ovarian cancer, colon cancer, colorectal cancer, breast cancer, lung cancer, myeloma, neuroblast-derived CNS tumor, monocytic leukemia, B cell-derived leukemia, T cell-derived leukemia, B cell-derived lymphoma, T cell-derived lymphoma, and mast cell-derived tumor, but is not limited thereto. Preferably, the cancer may be any one selected from the group consisting of ovarian cancer, lung cancer, B cell-derived lymphoma, and T cell-derived lymphoma.

To prepare the pharmaceutical composition of the present disclosure, the bispecific antibody of the present disclosure may be mixed with a pharmaceutically acceptable carrier and/or excipient. The pharmaceutical composition may be prepared in the form of a lyophilized preparation or aqueous solution. See, for example, Remington's Pharmaceutical Sciences and U.S. Pharmacopeia: National Formulary, Mack Publishing Company, Easton, PA (1984).

Acceptable carriers and/or excipients (including stabilizers) are non-toxic to subjects at dosages and concentrations used, and may include, are not limited to, buffers (for example, phosphate, citrate, or other organic acids); antioxidants (for example, ascorbic acid or methionine); preservatives (for example, octadecyldimethylbenzyl ammonium chloride; hexamethonium chloride; benzalkonium chloride, benzethonium chloride; phenol, butyl or benzyl alcohol; alkyl-parabens such as methyl- or propyl-paraben; catechol; resorcinol; cyclohexanol; 3-pentanol; and m-cresol); low molecular weight (not more than about 10 residues) peptides; proteins (for example, serum albumin, gelatin, or immunoglobulin); hydrophilic polymers (for example, polyvinylpyrrolidone); amino acids (for example, glycine, glutamine, asparagine, histidine, arginine, or lysine); monosaccharides, disaccharides, and other carbohydrates, for exmaple, glucose, mannose, or dextrin; chelating agents (for example, EDTA); sugars (for example, sucrose, mannitol, trehalose, or sorbitol); salt-forming counterions (for example, sodium); metal complexes (for example, Zn-protein complexes); and/or non-ionic surfactants (for example, TWEEN^{®}, PLURONICS^{®} or polyethylene glycol (PEG).

The pharmaceutical composition of the present disclosure may be formulated into a suitable form known in the art depending on its route of administration.

As used herein, the term "prophylactically or therapeutically effective amount" or "effective amount" refers to an amount of an active ingredient in a composition which is effective in preventing or treating cancer in a subject, the amount being sufficient to prevent or treat cancer at a reasonable benefit/risk ratio applicable to any medical treatment without causing any adverse effects. A level of the effective dose may be determined depending on factors including the patient's health status, type and severity of disease, activity of the drug, sensitivity to the drug, administration method, administration time, administration route and excretion rate, duration of treatment, and drugs used in combination or concurrently therewith, and other factors well known in the medical field. Here, taking all of the above factors into consideration, it is important to administer an amount that is a minimum amount and allows the maximum effect to be obtained with minimal or no adverse effects, and such an amount may be easily determined by those skilled in the art.

Specifically, the effective amount of the active ingredient in the pharmaceutical composition of the present disclosure may vary depending on age, gender, and body weight of the individual (patient), and is generally such that about 0.0001 mg to 100 mg, 0.001 to 50 mg, or 5 mg to about 50 mg per kg of body weight may be administered once a day or in divided doses several times a day. However, since the effective amount may increase or decrease depending on route and period of administration, severity of disease, gender, body weight, age, and the like, the scope of the present disclosure is not limited thereto.

Hereinafter, the present disclosure will be described in more detail by way of the following examples. The following examples are presented to help understand the present disclosure. These examples are not intended to limit a scope of the present disclosure in any way and should not be construed as limiting the present disclosure.

### Example 1. Preparation of bispecific antibody against CD47 and PD-L1

### Example 1.1. Sequences used for preparation of bispecific antibody against CD47 and PD-L1 and construction of vectors containing same

For preparation of an antibody having the structure illustrated in FIG. 1, a request for construction of vectors, which respectively contain the nucleotide sequence of the heavy chain (SEQ ID NO: 15) and the nucleotide sequence of the light chain (SEQ ID NO: 16) of this antibody, was made to Fusion Antibodies plc. (Belfast, Northern Ireland).

### Example 1.2. Preparation and purification of bispecific antibody against CD47 and PD-L1

Transient transfection of the vectors constructed in Example 1.1 was performed in Chinese hamster ovary (CHO) cell line. After batch culture, expressed antibodies were purified from cell culture supernatants. Quality control (QC) analysis was then completed on the purified antibodies to determine whether they met specified criteria as shown in Table 2.

**[Table 2]**

| **Specified criteria** | |
|---|---|
| **Purity:** | >95% |
| **Endotoxin (EU/mg):** | <0.25 |
| **Amount required (mg):** | 300 |
| **Concentration (mg/ml):** | >2 |
| **Others:** | Analytic SEC |

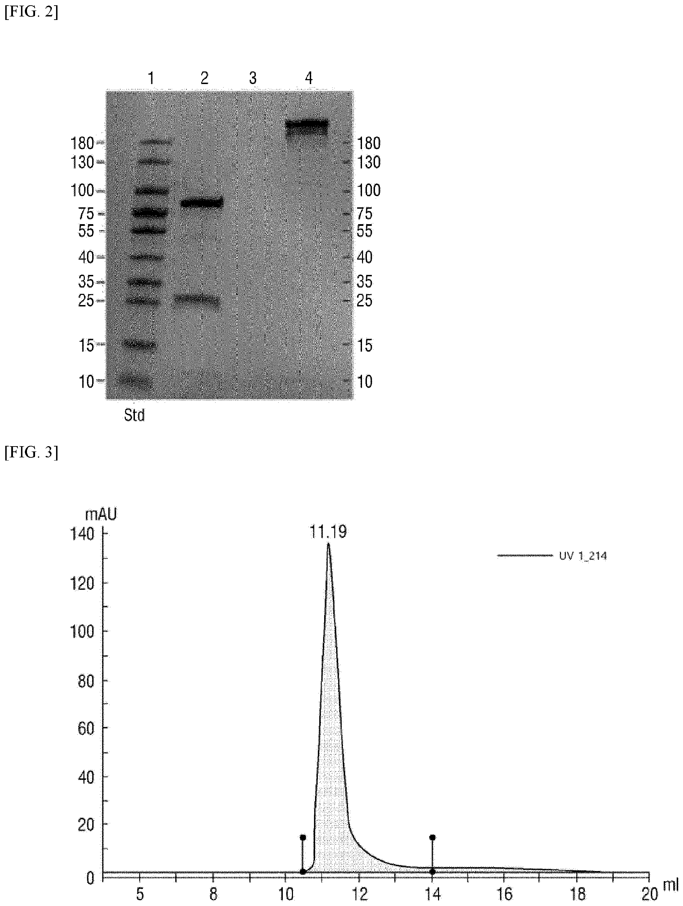

Specific procedures for the transfection, purification, and quality control analysis are as follows. The vectors constructed in Example 1.1 were cloned into pETE V2 (Fusion antibodies) which is a mammalian transient expression plasmid. The antibodies were expressed using a CHO-based transient expression system, and the resulting antibody-containing cell culture supernatants were purified by centrifugation and filtration. The antibodies were purified from the cell culture supernatants via affinity chromatography (using state-of-the-art AKTA chromatography equipment). The purified antibodies were buffer exchanged into phosphate buffered saline solution. Purity of the antibody was identified to be >95% as determined by reduced and denatured sodium dodecyl sulfate polyacrylamide gels (FIG. 2). In FIG. 2, the respective lanes are as shown in Table 3.

**[Table 3]**

| Lane number | Sample | Lot | Amount (mg) | Condition* |
|---|---|---|---|---|
| 1 | *PageRuler (Thermo Fisher)* | NA | NA | R/NR |
| 2 | Antibody of present example | 120520 | 2 | R/NR |
| 3 | Blank | NA | NA | NA |
| 4 | Antibody of present example | 120520 | 2 | R/NR |
| NA = Not available | | | | |
| *R = reducing, NR = non-reducing | | | | |

The antibodies were analyzed by size exclusion chromatography (SEC) to obtain a chromatogram (FIG. 3). The chromatogram showed one major peak (total area exceeding 95%). The integration results for the chromatogram shown in FIG. 3 are shown in Table 4 (based on the retention volume of control proteins (data not shown)). The peak corresponding to the monomer fraction was >98%.

**[Table 4]**

| **Peak** | **Retention (ml)** | **Area (ml*mAU)** | **% Area** |
|---|---|---|---|
| A | 11.19 | 83.87 | 100.00 |

Bacterial endotoxin levels were determined using the Endosafe^{®}-PTS system and the Endosafe^{®}-PTS cartridge (Charles River Laboratories). The antibody concentration was determined by measuring absorbance at 280 nm and performing calculation using a theoretical extinction coefficient for A280 of 1.0 mg/ml = 1.63. Details regarding the purified antibody product provided are summarized in Table 5.

**[Table 5]**

| Sample | Lot | Concentration (mg/ml) | Volume (ml) | Total (mg) | Monodispersity (%) | *Purity | Endotoxin (EU/mg) |
|---|---|---|---|---|---|---|---|
| Ab 1159.1 | 120520 | 1.83 | 822 | 516.06 | 100 | >95 | <0.2 |
| *Purity was determined by (relative band intensity) analysis of Coomassie-stained reduced and denatured SDS polyacrylamide gels. | | | | | | | |

From Table 5, it can be seen that the desired antibody was successfully expressed and purified to meet the specified criteria. The thus prepared bispecific antibody that specifically binds to CD47 and PD-L1 is hereinafter also referred to as "IMC-201."

The antibody prepared in this way has the following heavy and light chain variable and CDR sequences. The Fc region has the amino acid sequence of SEQ ID NO: 11 (see sequence listing).

**[Table 6]**

| | Domain | | Amino acid sequence | SEQ ID NO |
|---|---|---|---|---|
| Domain that specifically binds to CD47 | Heavy chain variable region | Full | Gln Val Gln Leu Val Gln Ser Gly Ala Glu Val Lys Lys Pro Gly Ala Ser Val Lys Val Ser Cys Lys Ala Ser Gly Tyr Thr Phe Thr Ser Tyr Tyr Met His Trp Val Arg Gln Ala Pro Gly Gln Gly Leu Glu Trp Met Gly Ile Ile Asn Pro Ser Gly Gly Ser Thr Ser Tyr Ala Gln Lys Phe Gln Gly Arg Val Thr Met Thr Arg Asp Thr Ser Thr Ser Thr Val Tyr Met Glu Leu Ser Ser Leu Arg Ser Glu Asp Thr Ala Val Tyr Tyr Cys Ala Arg Ser Thr Leu Trp Val Ser Glu Phe Asp Tyr Trp Gly Gln Gly Thr Leu Val Thr Val Ser Ser | 1 |
| | | CDR1 | **Gly Tyr Thr Phe Thr Ser Tvr** | 17 |
| | | CDR2 | **Asn Pro Ser Glv Glv Ser** | 19 |
| | | CDR3 | **Ser Thr Leu Trp Val Ser Glu Phe Asp Tyr** | 21 |
| | Light chain variable region | Full | Gln Ser Ala Leu Thr Gln Pro Ala Ser Val Ser Gly Ser Pro Gly Gln Ser Ile Ser Ile Ser Cys Thr Gly Thr Ser Ser Asp Val Gly Gly His Asn Tyr Val Ser Trp Tyr Gln Gln His Pro Gly Lys Ala Pro Lys Leu Met Ile Tyr Asp Val Arg Asn Arg Pro Ser Gly Val Ser Asn Arg Phe Ser Gly Ser Lys Ser Gly Ser Thr Ala Ser Leu Thr Ile Ser Gly Leu Gln | 3 |
| | | | Thr Glu Asp Glu Ala Asp Tyr Tyr Cys Asn Ser Tyr Thr Gly Ser Arg Thr Tyr Val Phe Gly Ser Gly Thr Lys Leu Thr Val Leu | |
| | | CDR1 | **Thr Gly Thr Ser SerAsp Val Gly Gly His Asn Tyr Val Ser** | 23 |
| | | CDR2 | **Asp Val Arg Asn Arg Pro Ser** | 25 |
| | | CDR3 | **Asn Ser Tyr Thr Gly Ser Arg Thr Tyr Val** | 27 |
| Domain that specifically binds to PD-L1 | Heavy chain variable region | Full | Gln Met Gln Leu Val Gln Ser Gly Ala Glu Val Lys Lys Pro Gly Ser Ser Val Lys Val Ser Cys Lys Ala Ser Gly Gly Thr Phe Ser Ser Tyr Ala Tyr Ser Trp Val Arg Gln Ala Pro Gly Gln Gly Leu Glu Trp Met Gly Gly Ile Ile Pro Ser Phe Gly Thr Ala Asn Tyr Ala Gln Lys Phe Gln Gly Arg Val Thr Ile Thr Ala Asp Glu Ser Thr Ser Thr Ala Tyr Met Glu Leu Ser Ser Leu Arg Ser Glu Asp Thr Ala Val Tyr Tyr Cys Ala Arg Gly Pro Ile Val Ala Thr Ile Thr Pro Leu Asp Tyr Trp Gly Gln Gly Thr Leu Val Thr Val Ser Ser | 5 |
| | | CDR1 | **Ser Tyr Ala Tyr Ser** | 29 |
| | | CDR2 | Gly Ile Ile Pro Ser Phe Gly Thr Ala Asn Tyr Ala Gln Lys Phe Gln | 31 |
| | | CDR3 | Gly Pro Ile Val Ala Thr Ile Thr Pro Leu Asp Tyr | 33 |
| | Light chain variable region | Full | Ser Tyr Val Leu Thr Gln Pro Pro Ser Val Ser Val Ala Pro Gly Lys Thr Ala Thr Ile Ala Cys Gly Gly Glu Asn Ile Gly Arg Lys Thr | 7 |
| | | | Val His Trp Tyr Gln Gln Lys Pro Gly Gln Ala Pro Val Leu Val Ile Tyr Tyr Asp Ser Asp Arg Pro Ser Gly Ile Pro Glu Arg Phe Ser Gly Ser Asn Ser Gly Asn Thr Ala Thr Leu Thr Ile Ser Arg Val Glu Ala Gly Asp Glu Ala Asp Tyr Tyr Cys Leu Val Trp Asp Ser Ser Ser Asp His Arg Ile Phe Gly Gly Gly Thr Lys Leu Thr Val Leu Gly Gln Pro Lys | |
| | | CDR1 | Gly Gly Glu Asn Ile Gly Arg Lys Thr Val His | 35 |
| | | CDR2 | **Tyr Asp Ser Asp Arg Pro Ser** | 37 |
| | | CDR3 | Leu Val Trp Asp Ser Ser Ser Asp His Arg Ile | 39 |

### Preparation Example 1. Preparation of anti-PD-L1 monoclonal antibody and anti-CD47 monoclonal antibody

The monoclonal antibodies prepared in this preparation example refer to homogeneous recombinant polypeptides that do not contain intrinsic contaminants.

First, the variable region sequences (SEQ ID NO: 1 and SEQ ID NO: 2) disclosed in US Patent No. 10,118,963 were used to prepare an anti-PD-L1 monoclonal antibody. The light and heavy chain antibody sequences were completed by linking human IgG1 constant region sequences, and the antibody was prepared using any expression system known in the art. Specifically, the antibody was prepared by performing transient expression using a recombinant expression vector, which contains a nucleic acid encoding the antibody, in a Chinese hamster ovary (CHO) cell line, and performing Protein A purification for the supernatant recovered from the host cells.

Next, the variable region sequences disclosed in U.S. Patent No. 10,035,855 (SEQ ID NO: 22 and SEQ ID NO: 8) were used to prepare an anti-CD47 monoclonal antibody. The light and heavy chain antibody sequences were completed by linking human IgG4 constant region sequences, and the antibody was prepared using any expression system known in the art. Specifically, the antibody was prepared by performing transient expression using a recombinant expression vector, which contains a nucleic acid encoding the antibody, in a Chinese hamster ovary (CHO) cell line, and performing Protein A purification for the supernatant recovered from the host cells.

The thus prepared anti-PD-L1 monoclonal antibody and anti-CD47 monoclonal antibody are hereinafter referred to as "IMC-001" and "IMC-002," respectively.

### Example 2. Evaluation of antigen binding ability and activity of IMC-201 (in vitro)

### Example 2.1. Identification of ligand binding ability by ELISA

First, binding capacity of IMC-201 to a single ligand (PD-L1) was measured by ELISA as follows. IMC-201 and IMC-001 were serially diluted (0.01 to 600 ng/ml, 3-fold dilution) and incubated with recombinant human PD-L1 pre-coated on plates at 75 ng/well. The HRP-conjugated anti-human IgG Fcy fragment specific antibody (Peroxidase AffiniPure Goat Anti-Human IgG, Fcy fragment specific (#109-035-098), Jackson IR Lab) was used for detection of IMC-201 and IMC-001.

Subsequently, binding capacity of IMC-201 to a single ligand (CD47) was measured by ELISA. IMC-201 and IMC-002 were serially diluted (0.01 to 600 ng/mL, 3-fold dilution) and incubated with recombinant human CD47 pre-coated on plates at 50 ng/well. The HRP-conjugated anti-human IgG Fcy fragment specific antibody was used for detection of IMC-201 and IMC-002.

Next, simultaneous binding capacity of IMC-201 to a dual ligand (PD-L1 and CD47) was measured by ELISA as follows. IMC-201, IMC-001, and IMC-002 were serially diluted (0.09 to 1800 ng/ml, 3-fold dilution), and respectively incubated with recombinant human CD47-fc pre-coated on plates at 50 ng/well per 100 µL. After washing, incubation was performed with recombinant human PD-L1-his at a concentration of 50 ng/well, and the HRP-conjugated anti-his fragment specific antibody was used for detection of IMC-201, IMC-001, and IMC-002.

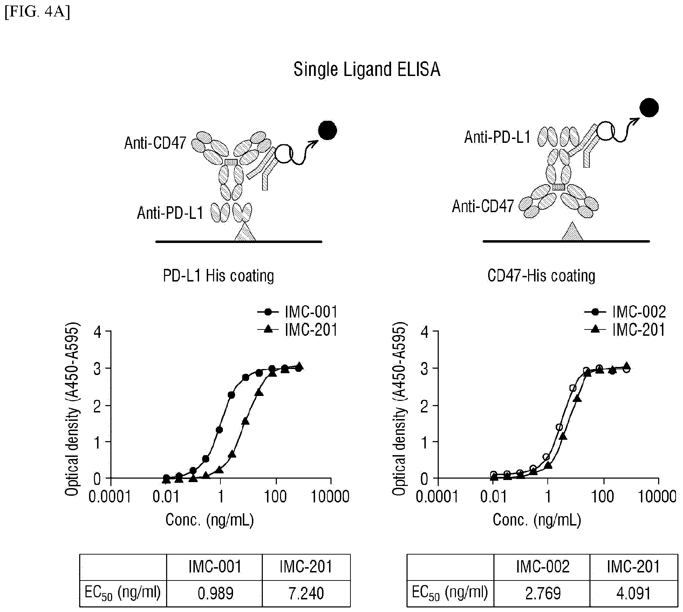

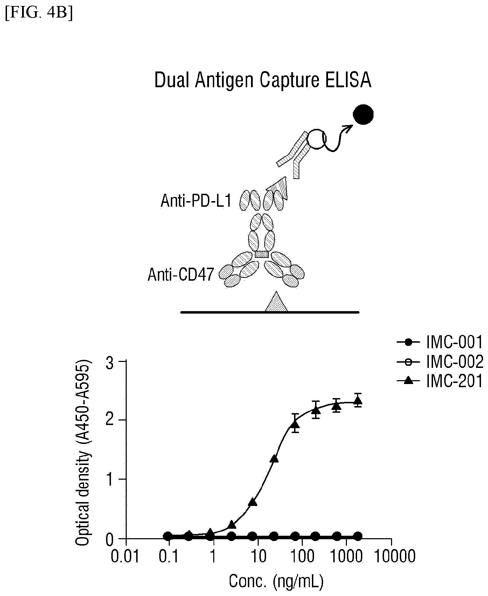

As a result, the EC₅₀ value (ng/ml) was measured to be 0.989 for IMC-001, which is a PD-L1 monoclonal antibody, and 7.240 for IMC-201 (left part in FIG. 4A). In addition, the EC₅₀ value was measured to be 2.769 for IMC-002, which is a CD47 monoclonal antibody, and 4.091 for IMC-201 (right part in FIG. 4A). Meanwhile, as a result of measuring the simultaneous binding capacity of IMC-201 to the dual ligand, it was identified that only the IMC-201 bispecific antibody specifically bound both CD47 and PD-L1 at the same time (EC₅₀: 17.380 ng/ml, FIG. 4B). In conclusion, it was identified that IMC-201 exhibited similar binding ability to the monoclonal antibodies IMC-001 and IMC-002.

### Example 2.2. Identification of binding ability to tumor cells

Binding ability of IMC-201 to tumor cells was measured as follows.

H1975 cell line with high PD-L1 expression (ATCC #CRL-5908) and Jurkat cell line with high CD47 expression (ATCC #TIB-152) were incubated with 3-fold diluted IMC-201, IMC-001, and IMC-002, respectively, at 4°C for 1 hour, and their binding strength was measured by flow cytometry (BD #LSRFortessa X-20).

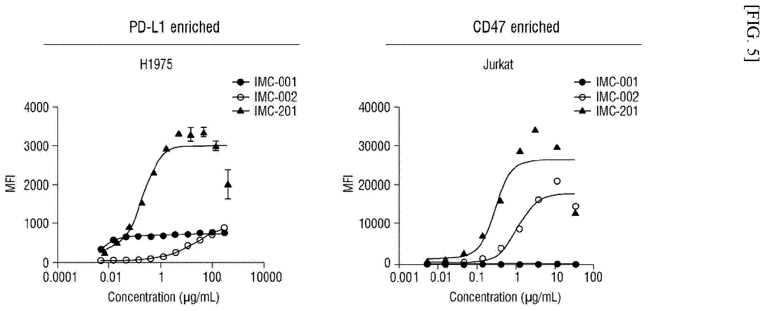

As a result, unlike the ELISA results, it was identified that IMC-201 had superior binding ability to the cells as compared with IMC-001 and IMC-002 (FIG. 5).

### Example 2.3. Identification of binding ability to knockout (KO) cell line

Binding capacity of IMC-201 to knockout stable cell lines was measured as follows.

PD-L1 (Sigma) KO cells, CD47 (Sigma) KO cells, and PD-L1 and CD47 double KO cells, obtained by performing knockout using MDA-MB-231 (ATCC #HTB-26) and CRISPR-Cas9, were incubated with 3-fold diluted IMC-201, IMC-001, and IMC-002, respectively, at 4°C for 1 hour. Their binding strength was measured by flow cytometry.

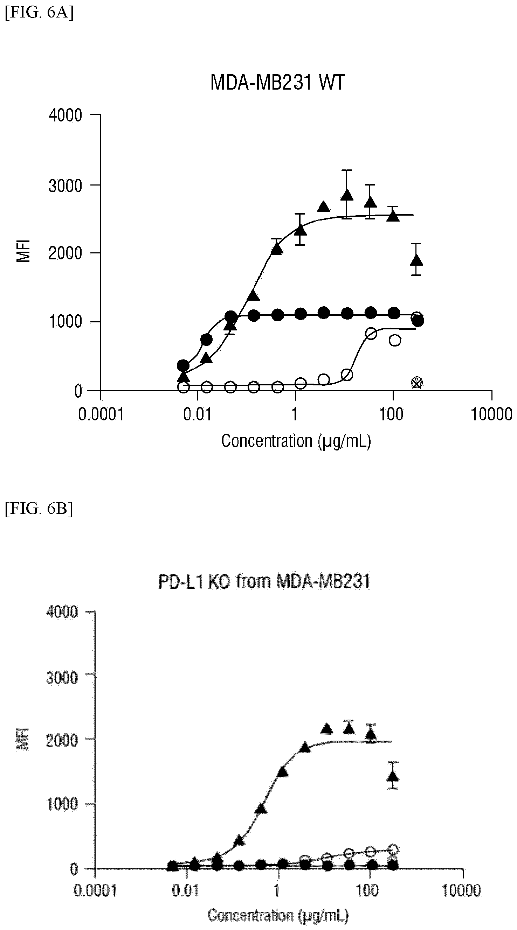

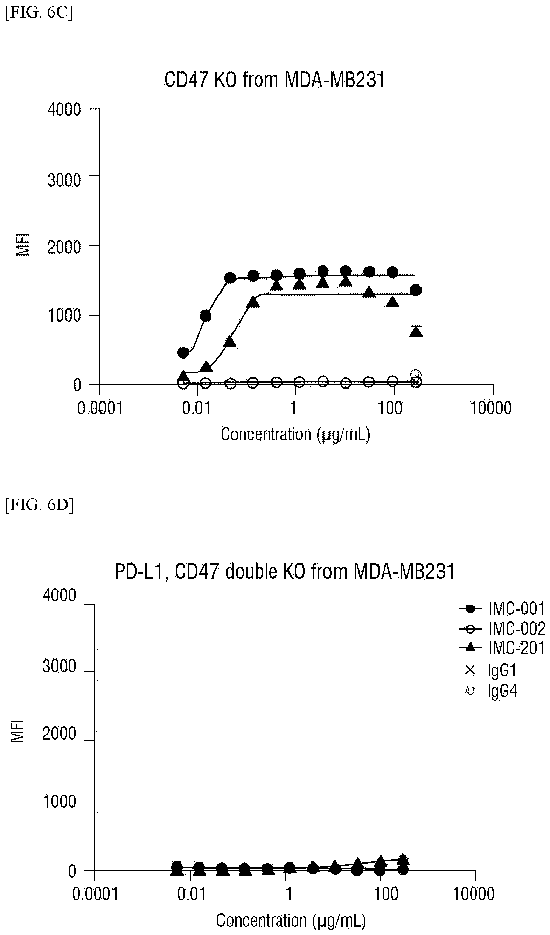

As a result, for the CD47 KO cells, the binding ability of IMC-201 was measured to be similar to that of IMC-001 which is its parent antibody, whereas for the PD-L1 KO cells, the binding ability of IMC-201 was measured to be significantly higher than that of IMC-002 which is its parent antibody. In conclusion, as identified in Example 2.2, it was found that the reason why IMC-201 had superior binding ability to tumor cells as compared with IMC-001 and IMC-002 is that the binding ability of IMC-201 to CD47 was much higher than that of the monoclonal antibody to CD47 (FIGS. 6A to 6D).

### Example 2.4. RBC binding capacity assay

Cell surface binding of IMC-201 to human red blood cells (RBCs) was measured as follows. Here, the anti-CD47 antibodies IMC-002 and Hu5F9 (which were prepared according to Liu J, et al. Pre-clinical development of a humanized anti-CD47 antibody with anti-cancer therapeutic potential. PLoS One. 2015;10(9):e0137345) were used as antibodies for comparison.

Human RBCs (5 × 10⁵ cells) obtained from healthy donors (n = 3) were incubated with 3-fold diluted IMC-201 or other antibodies for comparison (IMC-002 and Hu5F9), and their binding strength was determined using flow cytometry in which the determination was made by measuring geometric mean fluorescence intensity (MFI) of anti-human IgG (H+L) secondary antibody. As isotype controls, hIgG 1 (Sigma #15029) and hIgG4 (Biolegend #403702) were applied at a concentration of 300 µg/ml. The experiment was run in duplicate for each donor, and the average of MFI from the three donors was graphically represented using an error bar. The EC₅₀ value was calculated through nonlinear regression with GraphPad Prism 5.

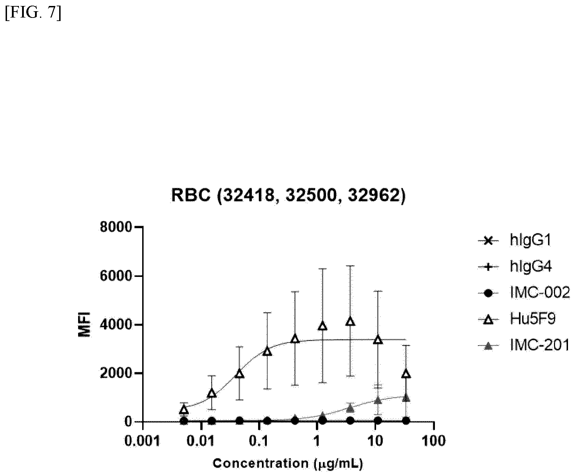

As a result, Hu5F9, which is an antibody for comparison, showed high RBC binding ability, whereas IMC-201 showed almost no RBC binding ability, which did not differ from the isotype controls and the monoclonal antibody IMC-002. Therefore, it was identified that IMC-201 had high binding ability to cancer cells (FIG. 5) and had low binding ability to RBCs (FIG. 7).

### Example 2.5. Hemagglutination assay

Human erythrocytes from healthy donors (n=3) were diluted 1/10 in 1X DPBS and incubated overnight at 37°C with the indicated concentrations of IMC-201 and other antibodies for comparison (IMC-002 and Hu5F9).

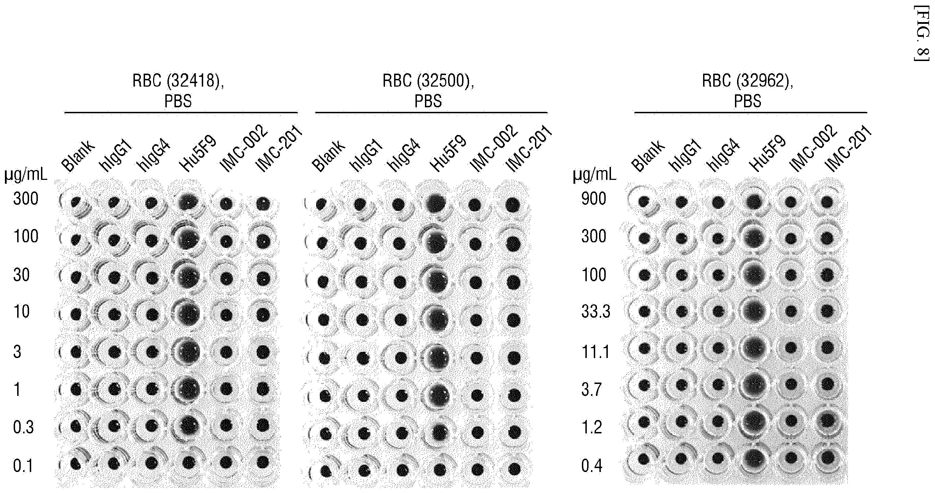

As a result, Hu5F9, which is an antibody for comparison, showed high hemagglutination, whereas IMC-201 showed almost no hemagglutination. A similar result was observed in a case of being compared with the blank and the isotype controls, and a similar result was also observed for the monoclonal antibody IMC-002. As identified in Example 2.4, it was found that IMC-201 showed little hemagglutination, as if reflecting its low binding ability to RBCs (FIG. 8).

### Example 2.6. Identification of phagocytosis

Monocyte-derived macrophages (MDM) were co-incubated with CFSE-labeled MDA-MB-231, H1975, Raji (ATCC#CCL-86), and SR786 (DSMZ#ACC369), respectively, at a 1:1 ratio in the presence of the antibodies for 2 hours. For MDA-MB-231 cells, IMC-002 and IMC-201 were applied at concentrations of 5 and 10 µg/ml; and for H1975 cells, IMC-002 and IMC-201 were applied at concentrations of 0.37, 1.11, and 3.33 µg/ml. For Raji cells, IMC-002 and IMC-201 were applied at concentrations of 0.04, 0.12, and 0.37 µg/ml; and for R786 cells, IMC-002 and IMC-201 were applied at concentrations of 0.01, 0.04, and 0.12 µg/ml.

Macrophage phagocytosis of tumor cells was assessed by flow cytometry in the presence of the test antibodies. Macrophages are defined as single living CD11b÷ cells. % phagocytosis is defined as a percentage of macrophages that are CFSE+.

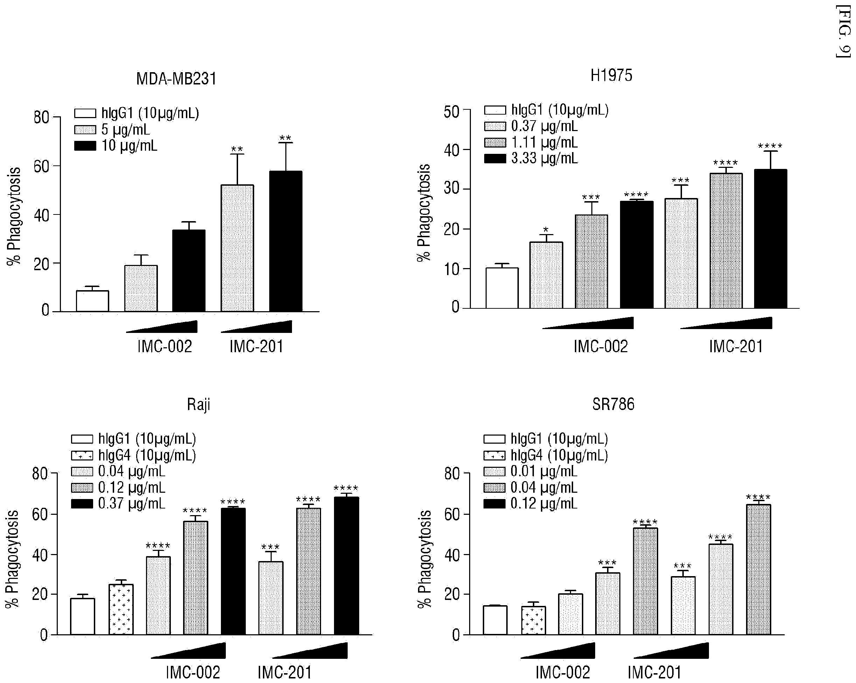

As a result, it was identified that phagocytosis of IMC-201 was equal to or better than that of IMC-002 for all four types of cells, and in particular, phagocytosis of IMC-201 was significantly high for MDA-MB-231 cells and H1975 cells (FIG. 9).

### Example 2.7. Evaluation of ADCC activity in tumor cells

ADCC activity was evaluated using the ADCC assay kit (Promega_G7018). The target cells, Raji, were coated on the plate on the day of testing, and the target cells, MDA-MB-231, were pre-coated on the plate overnight. The target cells for ADCC bioassay were co-incubated in the presence of the antibody (IMC-201, IMC-001, IMC-002, or Avelumab) at 4°C for 1 hour. Here, the antibody was serially diluted 4-fold for Raji cells and 7-fold for MDA-MB-231 cells. All antibodies, which did not bind to the target cells, were removed through washing, and then co-incubation was performed with effector cells in an incubator at 5% CO₂ for 6 hours. Luciferase was quantified using a Bio-Glo reagent.

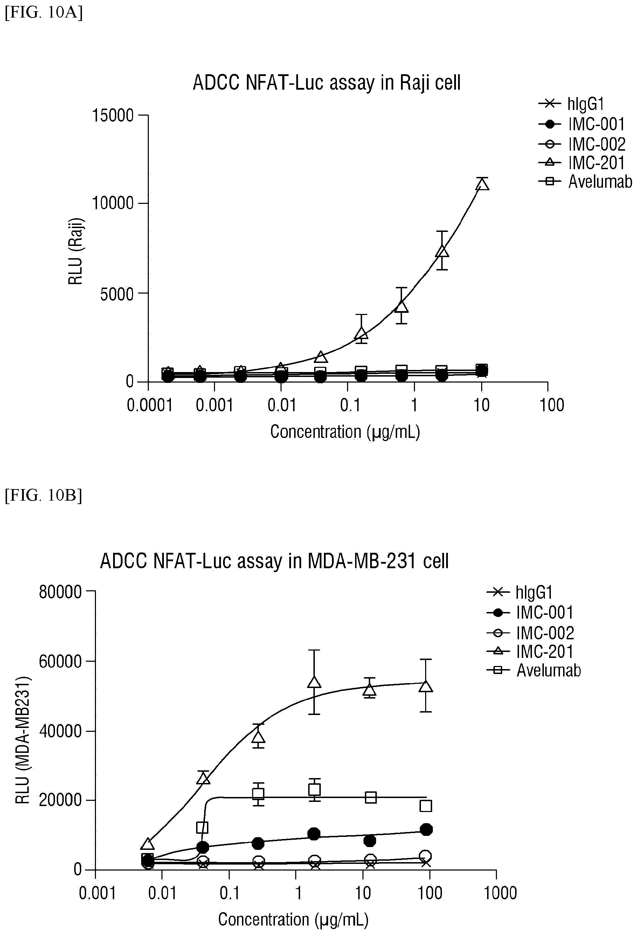

Consequently, a surprising result was observed for ADCC in which only IMC-201 showed an effect for the cells expressing CD47 (Raji) (FIG. 10A). In addition, IMC-201 showed the highest effect for the cells expressing both PD-L1 and CD47 (MDA-MB-231) (FIG. 10B).

### Example 2.8. Evaluation of ADCC activity of primary NK cells

ADCC of primary NK cells was evaluated by assessing CD107a degranulation. The target cells, MDA-MB-231, were pretreated with the antibodies (IMC-001, IMC-201) at concentrations of 0.1, 1, and 10 µg/ml for 30 minutes, and co-incubated with CD56+NK cells (effector cells) at a ratio of 1:1 (target cells:effector cells) for 4 hours at 37°C in an incubator at 5% CO₂. At the start of co-incubation, staining of CD107a was performed, and 1 hour later, GolgiStop^{®} protein transport inhibitor (containing monensin, purchased from BD Biosciences) was applied thereto. Then, staining of NK cell markers was performed and ADCC was evaluated as CD107a% in living NK cells (CD3-CD56+) using flow cytometry.

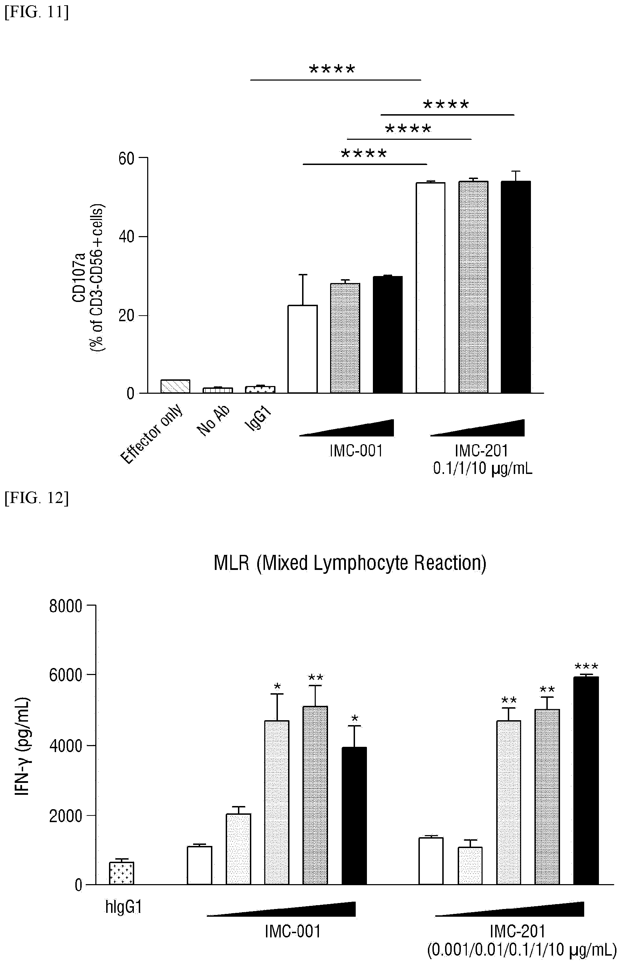

As a result, it was identified that activity of NK cells by IMC-201 was equivalent to that of competing drugs (data not shown) and was significantly superior to IMC-001 at all concentrations (FIG. 11). Increased NK cell activity indicates increased innate immunity activity caused by IMC-201.

### Example 2.9. Identification of T cell activity caused by antibodies (MLR analysis)

T cell activity caused by antibodies was identified through mixed lymphocyte reaction (MLR) analysis using dendritic cells and T cells. Monocyte-derived dendritic cells (moDC) were differentiated from CD14+ monocytes, and CD4+T cells were obtained from peripheral blood mononuclear cells (PBMCs) using an isolation kit (stem cell# 17952). The monocyte-derived dendritic cells and the CD4+T cells were co-incubated at a ratio of 1:10 in the presence of IMC-001 or IMC-201 (0.001 to 10 µg/mL, 10-fold dilution) for 5 days to obtain a cell culture medium. In the cell culture medium, expression of IFN-y was identified by ELISA.

As a result, it was identified that IFN-y increased depending on the concentration of IMC-201 (FIG. 12). IMC-201 showed similar efficacy to its parent antibody, IMC-001; and increased expression of IFN-y in the MLR analysis suggests possibility of suppressing cancer which results from increased cytotoxicity against cancer cells in a tumor microenvironment.

### Example 3. Identification of effect of IMC-201 (in vivo)

### Example 3.1. Cancer growth inhibition effect in breast cancer cell line xenograft model

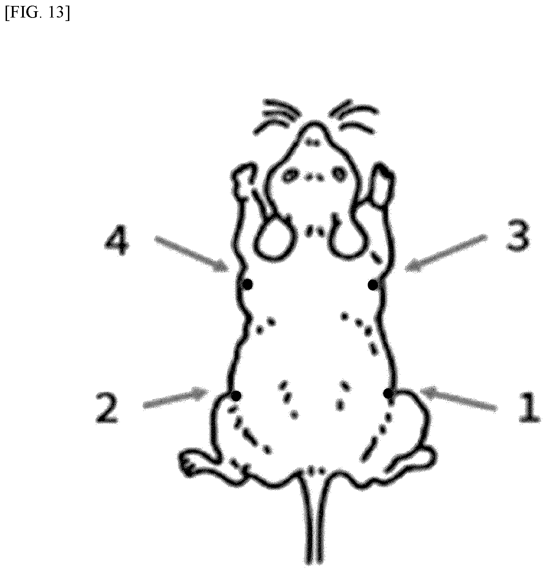

To study *in vivo* efficacy of IMC-201, MDA-MB-231 breast cancer cell line was thawed and cultured, and the cells were subcutaneously inoculated, at 1 × 10⁷ cells/200 µl, into each of 40 BALB/c nude mice lacking adaptive immune cells (purchased from Chemon) at site 1 shown in FIG. 13.

At day 17 after the cell inoculation, when the average tumor size reached 90.89 mm³, the mice were randomly assigned to five groups as shown in Table 7. Each group included 8 mice. Administration of the test substances began at D0. The test substance was administered intraperitoneally once a week (5 times in total).

Tumor volume and body weight were measured at D0, D4, D7, D11, D14, D18, D21, D25, D28, D32, D39, D46, and D53. The tumor volume was expressed in mm³ using the following equation: TV = 0.5 a × b² (where a and b are long and short diameters of tumor, respectively).

Data were expressed as mean ± standard error (Mean ± SEM). For comparison between the two groups, the independent-samples t test was performed. All data were analyzed with SPSS version 18.0. P<0.05 was considered statistically significant.

The efficacy study following transplantation of the breast cancer cell line was designed as follows:

**[Table 7]**

| Group | No. | Test article | Dose (mg/kg) | Administration method | Frequency | Administration period |
|---|---|---|---|---|---|---|
| G1 | 8 | IgG1 control | 21.4 | *i.p* | QW | 5w |
| G2 | 8 | IMC-001 | 2.1 | *i.p* | QW | 5w |
| G3 | 8 | IMC-002 | 2.1 | *i.p* | QW | 5w |
| G4 | 8 | IMC-001+IMC-002 | 2.1+2.1 | *i.p* | QW | 5w |
| G5 | 8 | IMC-201 | 3 | *i.p* | QW | 5w |
| Note: G, group; No, number of aminals; i.p, intraperitoneal; QW, once a week; | | | | | | |

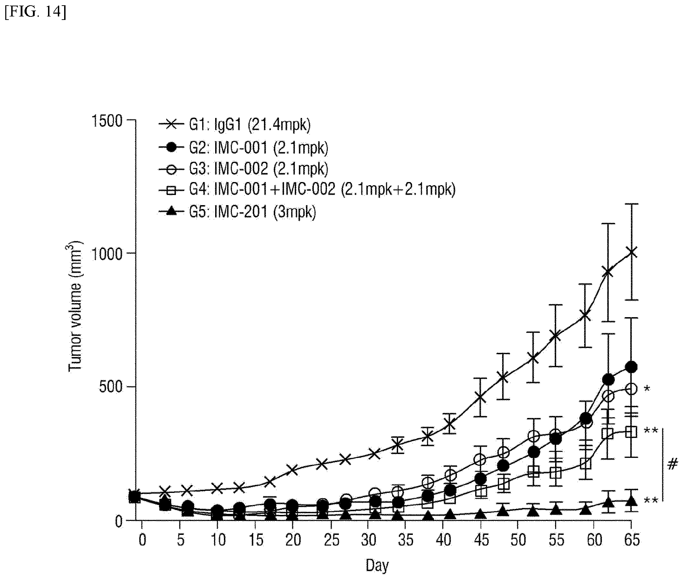

Regarding the results obtained by performing the efficacy study, the tumor growth inhibition, which is based on the tumor growth curve, is illustrated in FIG. 14.

From the results, the following was identified. At the efficacy study endpoint, day 65, the IMC-001 group (G2, 2.1 mpk, QW x 5w) showed no significant difference in tumor volume (TGItv = 42.70%) as compared with the isotype control (G1, IgG1 kappa, 21.4 mpk, QW x 5w). On the other hand, the IMC-002 group (G3, 2.1 mpk, QW x 5w) showed a significant difference in tumor volume (TGItv = 51.12%) as compared with the control (P<0.05*). In addition, the IMC-001 and IMC-002 combination-treated group (G4, 2.1 mpk + 2.1 mpk, QW x 5w) and the IMC-201-treated group (G5, 3 mpk, QW x 5w) also showed TGI values for tumor volume of 67.14% and 92.78%, respectively, indicating a significant difference (P<0.01**). In particular, the IMC-201-treated group showed the most significant tumor growth inhibition effect.

### Example 3.2. Cancer growth inhibition effect in colorectal cancer cell line xenograft model

The colorectal cancer cell line for studying the *in vivo* efficacy of IMC-201 is CT26-hPD-L1/hCD47. This cell line was produced by knocking out mouse PD-L1 and CD47 genes in CT26 WT cells (Kerafast, Cat No: ENH204) and inserting constitutively expressed human PD-L1 and CD47 genes therein, and was identified by flow cytometry.

The CT26-hPD-L1/hCD47 cells were thawed and passaged, and the cells were subcutaneously inoculated, at 2 × 10⁶ cells/100 µl, into each of 48 BALB/c-hPD-1/hSIRPα mice (purchased from GemPharmatech Co., Ltd) at site 3 shown in FIG. 13.

At day 7 after the cell inoculation, when the average tumor size reached 99.92 mm³, the mice were randomly assigned to five groups as shown in Table 8. Each group included 8 mice. The day of grouping was indicated as day 0 (D0). Administration of the test substance began at D0. The test substance was administered intraperitoneally twice a week (4 times in total). Tumor volume and body weight were measured on D0, D4, D7, D11, D14, D18, D21, D25, D28, D32, D39, D46, and D53. The tumor volume was expressed in mm³ using the following equation: TV = 0.5 a × b² (where a and b are long and short diameters of tumor, respectively).

Data were expressed as mean ± standard error (Mean ± SEM). For comparison between the two groups, the independent-samples t test was performed. All data were analyzed with SPSS version 18.0. P<0.05 was considered statistically significant.

The efficacy study following transplantation of the colorectal cancer cell line was designed as follows:

**[Table 8]**

| Group | No. | Test article | Dose (mg/kg) | Administration method | Frequency | Administration period |
|---|---|---|---|---|---|---|
| G1 | 8 | Isotype control | 21.4 | *i.p* | BIW | 2w |
| G2 | 8 | IMC-001 | 2.1 | *i.p* | BIW | 2w |
| G3 | 8 | IMC-002 | 2.1 | *i.p* | BIW | 2w |
| G4 | 8 | IMC-001+IMC-002 | 2.1 | *i.p* | BIW | 2w |
| G5 | 8 | IMC-201 | 3 | *i.p* | BIW | 2w |
| Note: G, group; No, number of animals; i.p, intraperitoneal; BIW, twice a week; | | | | | | |

Among the test articles, the isotype control is IgG1 kappa (Crownbio).

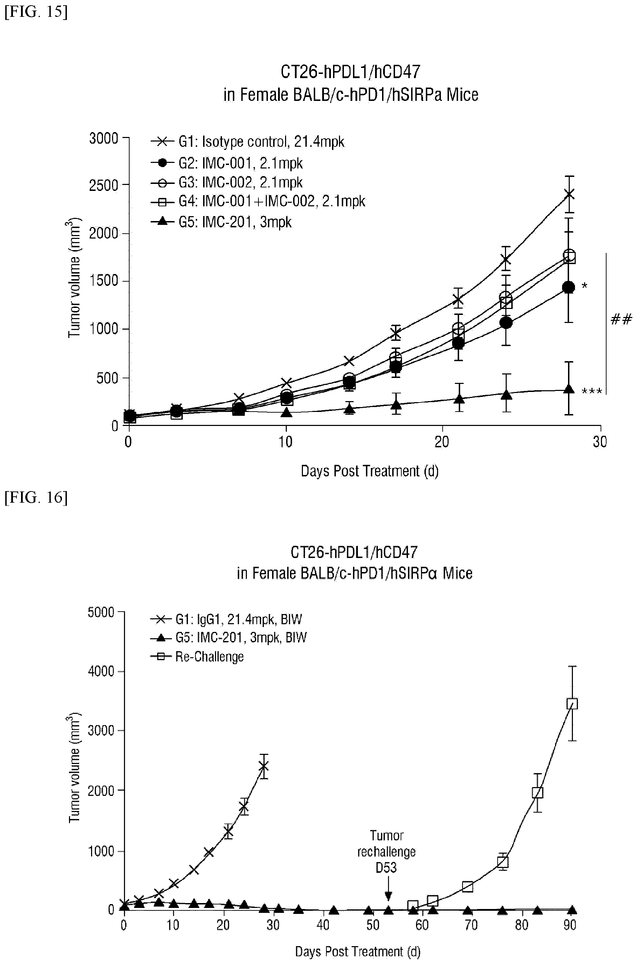

Regarding the results obtained by performing the efficacy study, the tumor growth inhibition, which is based on the tumor growth curve, is illustrated in FIG. 15.

From the results, the following was identified. At the efficacy study endpoint, G3 (IMC-002 administration group, 2.1 mpk, BIW X 2w) showed a non-significant difference in tumor volume (TGItv D28 = 27.08%) and tumor weight (TGItw = 21.11%) as compared with the isotype control G1 (IgG1 kappa, 21.4 mpk, BIW X 2w). In addition, G4 (IMC-001+IMC-002 combination-treated group, 2.1 mpk + 2.1 mpk, BIW X 2w) showed a non-significant difference in tumor volume (TGItv D28 = 27.37%) and tumor weight (TGItw = 26.93%) as compared with G1. On the other hand, the IMC-001-treated group (G2, 2.1 mpk, BIW X 2w) showed a significant difference in tumor volume (TGItv D28 = 38.60%) and tumor weight (TGItw = 41.43%) as compared with G1 (P< 0.05*). Moreover, G5 (IMC-201, 3 mpk, BIW X 2w) showed high statistical significance in tumor volume (TGItv D28 = 83.57%) and tumor weight (TGItw = 82.98%) (P<0.001***). No abnormal body weight changes were observed in all treatment groups. For administration of IMC-201, a significant level of cancer growth inhibition was observed and a higher level of cancer growth inhibition effect was identified than the groups treated with its parent antibodies IMC-001 and IMC-002 alone or in combination.

### Example 3.3. Re-challenge study

A re-challenge study was performed, at day 53 after the grouping for efficacy study in Example 3.2, by subcutaneously inoculating the CT26-hPD-L1/hCD47 tumor cells at 2×10⁶ cells/100 µL into each mouse at site 4 shown in FIG. 13. The endpoint of the re-challenge study was estimated to be day 88. At the endpoint of the re-challenge study, the mice and their tumors were photographed, and tumor weights were measured. Regarding the results obtained by performing the re-challenge study, changes in the average tumor volume over time for the various groups are shown in FIG. 16.

From the results, the following was identified. At the study endpoint, day 88, G1 (BALB/c-hPD-1/hSIRPα mice) and G5 (3 mpk (BIW X 2w) IMC-201-treated mice with tumor regression) showed the average tumor volumes of 3233.74 mm³ and 0 mm³, respectively. As compared with the control (G1), G5 group (3 mpk (BIW X 2w) IMC-201 treated mice with tumor regression) showed a significant tumor inhibition effect. Tumor formation occurred in the mice (G1) that did not receive the IMC-201 antibody at day 53 after the grouping for efficacy study. On the other hand, it was identified that for the mice (G5) whose tumor was suppressed by the IMC-201 antibody, no tumor was generated even without re-administration of the antibody after re-transplanting cancer cells on the opposite side of the previously transplanted site, in which complete response (CR) was observed in 6 mice.

From the results, it was identified that IMC-201 (3 mpk) had an inhibitory effect on tumor re-challenge due to generation of memory T cells in the re-challenge study using the same tumor.

## Claims

1. A bispecific antibody, comprising a first antigen binding domain that specifically binds to CD47, and a second antigen binding domain that specifically binds to PD-L1,
wherein the first antigen binding domain comprises a heavy chain variable region and a light chain variable region, the heavy chain variable region comprising CDR1 that comprises the amino acid sequence of SEQ ID NO: 17, CDR2 that comprises the amino acid sequence of SEQ ID NO: 19, and CDR3 that comprises the amino acid sequence of SEQ ID NO: 21, and the light chain variable region comprising CDR1 that comprises the amino acid sequence of SEQ ID NO: 23, CDR2 that comprises the amino acid sequence of SEQ ID NO: 25, and CDR3 that comprises the amino acid sequence of SEQ ID NO: 27, and
the second antigen binding domain comprises a heavy chain variable region and a light chain variable region, the heavy chain variable region comprising CDR1 that comprises the amino acid sequence of SEQ ID NO: 29, CDR2 that comprises the amino acid sequence of SEQ ID NO: 31, and CDR3 that comprises the amino acid sequence of SEQ ID NO: 33, and the light chain variable region comprising CDR1 that comprises the amino acid sequence of SEQ ID NO: 35, CDR2 that comprises the amino acid sequence of SEQ ID NO: 37, and CDR3 that comprises the amino acid sequence of SEQ ID NO: 39.

2. The bispecific antibody of claim 1, wherein the first antigen binding domain comprises a heavy chain variable region of SEQ ID NO: 1 and a light chain variable region of SEQ ID NO: 3.

3. The bispecific antibody of claim 1, wherein the second antigen binding domain comprises (i) a heavy chain variable region of SEQ ID NO: 5 and a light chain variable region of SEQ ID NO: 7 or (ii) a single chain variable fragment (scFv) of SEQ ID NO: 9.

4. The bispecific antibody of claim 1, wherein the bispecific antibody comprises an Fc domain, a first antibody fragment comprising the antigen binding domain that specifically binds to CD47, and a second antibody fragment comprising the antigen binding domain that specifically binds to PD-L1.

5. The bispecific antibody of claim 4, wherein the first antibody fragment is an Fab fragment, and the second antibody fragment is a single chain variable fragment (scFv).

6. The bispecific antibody of claim 4, wherein the second antibody fragment is fused, via a peptide linker, to the C-terminus of the Fc domain.

7. The bispecific antibody of claim 1, wherein the bispecific antibody comprises an Fc domain, two Fab fragments each comprising the antigen binding domain that specifically binds to CD47, and two scFv fragments each comprising the antigen binding domain that specifically binds to PD-L1.

8. The bispecific antibody of claim 1, wherein the bispecific antibody comprises a single chain variable fragment (scFv) comprising the antigen binding domain that specifically binds to PD-L1.

9. The bispecific antibody of claim 1, wherein the bispecific antibody comprises an Fab fragment comprising the antigen binding domain that specifically binds to CD47.

10. The bispecific antibody of claim 1, wherein the bispecific antibody comprises an Fc region derived from the heavy chain constant region (CH) of IgG1.

11. The bispecific antibody of claim 10, wherein the Fc region comprises heavy chain constant regions CH1, CH2, and CH3 derived from IgG1, and CH3 contains amino acid substitutions of E239D and M241L with respect to SEQ ID NO: 11.

12. The bispecific antibody of claim 10, wherein the Fc region acts on effector cells to exhibit an immune activation effect.

13. The bispecific antibody of claim 10, wherein the bispecific antibody comprises an antibody fragment comprising the antigen binding domain that specifically binds to PD-L1, and the antibody fragment is fused to the C-terminus of the Fc domain via a peptide linker.

14. The bispecific antibody of claim 8, wherein the single chain variable fragment (scFV) comprises the amino acid sequence of SEQ ID NO: 9.

15. The bispecific antibody of claim 10, wherein the Fc region comprises the amino acid sequence of SEQ ID NO: 11.

16. The bispecific antibody of claim 1, wherein the bispecific antibody specifically binds cancer cells that express PD-L1, CD47, or both.

17. The bispecific antibody of claim 1, for use in prevention or treatment of a carcinoma selected from the group consisting of breast cancer, lung cancer, B cell-derived lymphoma, and T cell-derived lymphoma.

18. A pharmaceutical composition for prevention or treatment of cancer, comprising:
the bispecific antibody of any one of claims 1 to 17.

19. The pharmaceutical composition of claim 18, wherein the cancer is selected from the group consisting of ovarian cancer, colon cancer, breast cancer, lung cancer, myeloma, neuroblast-derived CNS tumor, monocytic leukemia, B cell-derived leukemia, T cell-derived leukemia, B cell-derived lymphoma, T cell-derived lymphoma, and mast cell-derived tumor.

20. The pharmaceutical composition of claim 18, wherein the cancer is selected from the group consisting of breast cancer, lung cancer, B cell-derived lymphoma, and T cell-derived lymphoma.

21. A pharmaceutical composition for use in inhibition of tumor cell growth, comprising:
the bispecific antibody of any one of claims 1 to 17.

22. The pharmaceutical composition of claim 18, wherein the bispecific antibody is used or administered in combination with chemotherapy, radiation therapy, and/or another cancer immunotherapeutic agent.

23. A use of the bispecific antibody of any one of claims 1 to 17, for prevention or treatment of various carcinomas including breast cancer, lung cancer, B cell-derived lymphoma, and T cell-derived lymphoma.

24. A method for preventing or treating cancer, comprising:
administering an effective amount of the bispecific antibody of any one of claims 1 to 17 to a subject in need of prevention or treatment of cancer.

25. A method for inhibiting growth of tumor cells in a subject, comprising:
administering, to the subject having tumor cells, an effective amount of the bispecific antibody of any one of claims 1 to 17.

26. A polynucleotide, encoding the bispecific antibody of any one of claims 1 to 17.

27. A host cell, comprising the polynucleotide of claim 26.

28. A method for preparing the bispecific antibody of any one of claims 1 to 17, comprising:
culturing the host cell of claim 27 under a condition suitable for expression of the bispecific antibody; and
collecting the bispecific antibody from the culture.
